# EUROPEAN PATENT APPLICATION

(11) **EP 4 124 341 A1**
(43) Date of publication of application: **01.02.2023**
(21) Application number: 22187311.0
(22) Date of filing: 27.07.2022
(51) Int. Cl.: A61K 35/12, A61K 35/28, B01D 61/00

(54) **EXOSOME ENRICHMENT BY ULTRAFILTRATION**

(30) Priority: 27.07.2021 US 202163203662 P
(71) Applicant: National Yang Ming Chiao Tung University, Hsinchu City, 300 (TW)
(72) Inventor: LEE, OSCAR KUANG-SHENG, 112 TAIPEI CITY (TW); HO, JENNIFER HUI-CHUN, SAN DIEGO, 92130 (US); WU, HAO-HSIANG, 112 TAIPEI CITY (TW)
(74) Representative: Lavoix

(57) **Abstract**

The present disclosure provides an exosome composition comprising a population of enriched exosomes. The exosome composition is directly obtained by isolation and ultrafiltration rather than by formulation. The present disclosure also provides a method for producing the exosome composition.

## Description

### Field of the Invention

The present disclosure relates to an exosome composition comprising enriched exosomes and production thereof.

### Background of the Invention

Exosomes are membrane-bound extracellular vesicles (EVs) that are produced in the endosomal compartment of most eukaryotic cells. Exosomes generally have a size range of -40 nm to 160 nm (average ∼100 nm) in diameter with an endosomal origin. Their surface consists of a lipid bilayer from the donor cell's cell membrane, and they contain cytosol from the cell that produced the exosome, and exhibit membrane proteins from the parental cell on the surface. Exosomes were initially thought to be a mechanism for removing unneeded proteins. Exosomes can bind to a cell surface receptor in a similar way to cell-to-cell interaction. Also, exosomes can attach to the cell membrane and give the cells new receptors and properties. Thus, exosomes can fuse with target cells and exchange membrane proteins and cytosol between two cell types. It is known that they mediate communication between cells and facilitate transfer of proteins. It is also known that exosome cargo includes a wide range of signaling factors, and these signaling factors are specific for cell types and regulated differently depending on secretory cells' environment. Exosomes, as natural intercellular information carriers, have a huge application potential in the field of drug carriers due to their natural material-transporting property, relatively smaller molecular structure and excellent biocompatibility.

### Summary of the Invention

One aspect of the present disclosure relates to an exosome composition comprising enriched exosomes having specific distribution in size and molecular weight.

In one embodiment, the exosome composition comprises a population of enriched exosomes in a concentration greater than about 1 × 10⁹ exosomes/mL, wherein the exosome composition is directly obtained by isolation and ultrafiltration or ultracentrifugation rather than by formulation.

In a further embodiment, the concentration of the exosomes in the composition is greater than about 1 × 10¹⁰ exosomes/mL. In some embodiments, the concentration of the exosomes in the composition ranges from about 1 × 10¹⁰ exosomes/mL to about 1 × 10¹⁵ exosomes/mL, about 1 × 10¹⁰ exosomes/mL to about 1 × 10¹⁴ exosomes/mL, about 1 × 10¹⁰ exosomes/mL to about 1 × 10¹³ exosomes/mL, about 1 × 10¹⁰ exosomes/mL to about 1 × 10¹² exosomes/mL, about 1 × 10¹⁰ exosomes/mL to about 1 × 10¹¹ exosomes/mL or about 1 × 10¹⁰ exosomes/mL to about 5 × 10¹⁰ exosomes/mL.

In one embodiment, the exosome composition comprises a population of enriched exosomes having a mean particle size from about 135 nm to about 150 nm or about 138 nm to about 148 nm.

In one embodiment, the population of enriched exosomes described herein comprises exosomes having molecular weight greater than about 3 kDa, about 10 kDa, about 50 kDa or about 100 kDa.

In one embodiment, the population of enriched exosomes described herein comprises greater than about 75%, greater than about 80%, greater than about 85%, greater than about 88% or greater than about 90% exosomes having a particle size less than about 200 nm. In some embodiments, the population of enriched exosomes described herein comprises about 75% to about 95%, about 80% to about 95%, about 85% to about 95%, about 75% to about 93%, about 80% to about 93%, about 85% to about 93% or about 85% to about 92% of exosomes having a particle size less than about 200 nm.

In one embodiment, the population of enriched exosomes described herein comprises greater than about 10%, greater than about 12% or greater than about 15% exosomes having a particle size less than 100 nm. In some embodiments, the population of enriched exosomes described herein comprises about 10% to about 25%, about 10% to about 20%, about 12% to about 25%, about 12% to about 20%, about 15% to about 25%, about 15% to about 20%, about 16% to about 25% or about 16% to about 20% of exosomes having a size less than about 100 nm.

In some embodiments, the population of enriched exosomes described herein comprises exosomes with a molecular weight greater than about 3 kDa, which have one or more of the following characteristics:
a concentration greater than 1 × 10¹⁰ exosomes/mL;
about 90% to about 93% of exosomes having a particle size less than about 200 nm;
about 16% to about 20% of exosomes having a particle size less than about 100 nm; and
a mean particle size from about 135 nm to about 145 nm.

In further embodiments, the concentration is greater than about 2 × 10¹⁰ exosomes/mL or about 3 × 10¹⁰ exosomes/mL (preferably about 3.2 10¹⁰ exosomes/mL); about 92% of exosomes have a particle size less than about 200 nm; about 17% to about 19% (preferably about 18.6 × 10¹⁰ exosomes/mL) of exosomes have a particle size less than about 100 nm; and/or a mean particle size ranges from about 137 nm to about 142 nm (preferably about 140 nm).

In some embodiments, the population of enriched exosomes described herein comprises exosomes with a molecular weight greater than about 10 kDa, which have one or more of the following characteristics:
a concentration greater than 2 × 10¹⁰ exosomes/mL;
about 90% to about 93% of exosomes having a particle size less than about 200 nm;
about 14% to about 18% of exosomes having a particle size less than about 100 nm; and
a mean particle size from about 138 nm to about 148 nm.

In further embodiments, the concentration is greater than about 2.5 × 10¹⁰ exosomes/mL, about 3.0 × 10¹⁰ exosomes/mL, about 3.5 × 10¹⁰ exosomes/mL or about 4.0 × 10¹⁰ exosomes/mL (preferably about 4.2 × 10¹⁰ exosomes/mL); about 91% of exosomes have a particle size less than about 200 nm; about 15% to about 17% (preferably about 16.27 × 10¹⁰ exosomes/mL) of exosomes have a particle size less than about 100 nm; and/or a mean particle size ranges from about 140 nm to about 145 nm (preferably about 143.7 nm).

In some embodiments, the population of enriched exosomes described herein comprises exosomes with a molecular weight greater than about 50 kDa, which have one or more of the following characteristics:
a concentration greater than 1.0 × 10¹⁰ exosomes/mL;
about 85% to about 90% of exosomes having a particle size less than about 200 nm;
about 15% to about 25% of exosomes having a particle size less than about 100 nm; and
a mean particle size from about 140 nm to about 150 nm.

In further embodiments, the concentration is greater than about 1.0 × 10¹⁰ exosomes/mL (preferably about 1.44 × 10¹⁰ exosomes/mL); about 88.86% of exosomes have a particle size less than about 200 nm; about 18% to about 20% (preferably about 19.21 × 10¹⁰ exosomes/mL) of exosomes have a particle size less than about 100 nm; and/or a mean particle size from about 142 nm to 148 nm (preferably about 145 nm).

In some embodiments, the population of enriched exosomes described herein comprises exosomes with a molecular weight greater than about 100 kDa, which have one or more of the following characteristics:
a concentration greater than 1.0 × 10¹⁰ exosomes/mL;
about 88% to about 92% of exosomes having a particle size less than about 200 nm;
about 15% to about 20% of exosomes having a particle size less than about 100 nm; and
a mean particle size from about 138 nm to about 145 nm.

In further embodiments, the concentration is greater than about 1.2 × 10¹⁰ exosomes/mL (preferably about 1.48 × 10¹⁰ exosomes/mL); about 90.93% of exosomes have a particle size less than about 200 nm; about 16% to about 19% (preferably about 18.7 × 10¹⁰ exosomes/mL) of exosomes have a particle size less than about 100 nm; and/or a mean particle size ranges from about 140 nm to about 144 nm (preferably about 142.2 nm).

In one embodiment, the exosomes described herein are derived from stem cells. In some embodiments, the stem cells are embryonic stem cells (ESCs), mesenchymal stem cells (MSCs), hematopoietic stem-cell transplantation (HSCT) or induced pluripotent stem cells (iPS cells or iPSCs).

Another aspect of the present disclosure relates to a method for producing such exosome population.

In one embodiment, the present disclosure provides a method for producing the exosome composition described herein, comprising the steps of:
providing a desired amount of cells in a medium;
centrifuging the medium to remove cell debris and then filtrating the resulting supernatant to remove cell apoptotic bodies and microvesicles;
ultrafiltrating the resulting supernatant with a membrane having a molecular weight cutoff of about 3 kDa to about 100 kDa; and
precipitating exosomes with polymer-based precipitation to obtain the exosome composition comprising enriched exosomes.

In one embodiment, the cells used in the method are stem cells. In some embodiments, the stem cells are embryonic stem cells (ESCs), mesenchymal stem cells (MSCs), hematopoietic stem-cell transplantation (HSCT) or induced pluripotent stem cells (iPS cells or iPSCs)..

In some embodiments of the disclosure, the ultrafiltration with a membrane having a molecular weight cutoff is performed via concentrator spin columns.

In one embodiment, the present disclosure provides a method for producing the exosome composition described herein, comprising the steps of:
providing a desired amount of cells in a medium;
centrifuging the medium to remove cell debris and then filtrating the resulting supernatant to remove cell apoptotic bodies and microvesicles; and
ultracentrifugating the resulting supernatant at about 80,000 g to about 12,000 g for about 50 minutes to about 90 minutes to obtain the exosome composition comprising enriched exosomes.

In one embodiment, the ultracentrifugation ranges from about 80,000 g to about 12,000 g, about 85,000 g to about 11,500 g, about 90,000 g to about 11,000 g, or about 95,000 g to about 10,500 g, for about 50 minutes to about 90 minutes, about 55 minutes to about 85 minutes, about 60 minutes to about 80 minutes, or about 65 minutes to about 75 minutes.

### Brief Description of the Drawings

FIG. 1 shows the results of time consuming of ultrafiltration.
FIG. 2 shows the results of nanoparticle tracking analysis in Example 1.
FIG. 3 shows parameter of nanoparticle tracking analysis. Mean size, D90, and D10 are shown in 3 kDa, 10 kDa, 50 kDa, and 100 kDa ultrafiltration.
FIG. 4 shows expression of exosome markers. Alix is an intra-exosomal protein. CD81 is a surface protein of exosomes and decreases in 50 kDa and 100 kDa compared to 3 kDa.

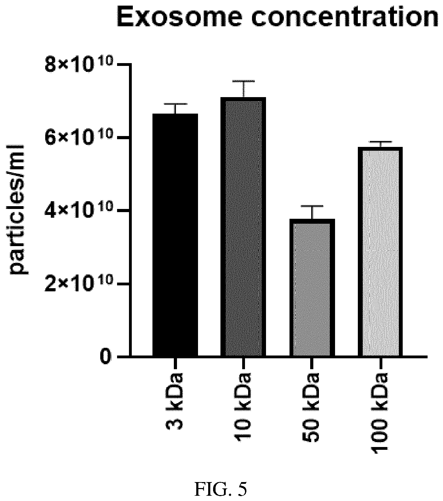

FIG. 5 shows particle concentration. Particle concentration was estimated. Particle concentration decreased in 50 kDa and 100 kDa compared to 3 kDa via nanoparticle tracing analysis.
FIG. 6 shows the transmission electron microscopy (TEM) analysis in UCMSCs-derived exosomes.
FIG. 7 shows the results of nanoparticle tracking analysis in Examples 1 and 3.
FIG. 8 shows parameter of nanoparticle tracking analysis in Examples 1 and 3.
FIG. 9 shows Particle concentration and expression of exosome markers in Examples 1 and 3.

### Detailed Description of the Invention

Unless otherwise specified or indicated by context, the terms "a," "an," and "the" mean "one or more."

As used herein, "about" will be understood by persons of ordinary skill in the art and will vary to some extent with the context in which they are used. If there are uses of the term which are not clear to persons of ordinary skill in the art given the context in which they are used, "about" will mean plus or minus 10% of the particular term.

The term "exosome" as used herein refers to any extracellular vesicle derived from any body fluid from a human or an animal (e.g., blood), any extracellular vesicle derived from human or animal cell lines, cell cultures, and primary cultures not limited to autologous exosomes, universal donor exosomes, allogenic exosomes, and modified exosomes.

The term "formulation" refers to a preparation which is in such form as to permit the biological activity of the active agent to be effective.

As used herein, the term "stem cell" refers to a cell in an undifferentiated or partially differentiated state that has the property of self-renewal and has the developmental potential to naturally differentiate into a more differentiated cell type, without a specific implied meaning regarding developmental potential (i.e., totipotent, pluripotent, multipotent, etc.). By self-renewal is meant that a stem cell is capable of proliferation and giving rise to more such stem cells, while maintaining its developmental potential. Accordingly, the term "stem cell" refers to any subset of cells that have the developmental potential, under particular circumstances, to differentiate to a more specialized or differentiated phenotype, and which retain the capacity, under certain circumstances, to proliferate without substantially differentiating.

As used herein, the term "derived from" shall be taken to indicate that a particular sample or group of samples has originated from the species specified, but has not necessarily been obtained directly from the specified source.

Exosomes by themselves or as vehicles for the delivery of drug payload(s) are being actively explored as therapeutic agents. In contrast to liposomes, injected exosomes are efficient at entering other cells and can deliver a functional cargo with minimal immune clearance and exhibit good tolerance upon exogenous administration.

The heterogeneity of exosomes is likely reflective of their size, content, functional impact on recipient cells, and cellular origin. Size inequality could be due to uneven invagination of the limiting membrane of the multivesicular bodies (MVB), resulting in distinct total content of fluid and solids, or isolation methods that include other EVs. Refined fractionation methods involving EVs revealed that exosomes may contain subpopulations defined by a distinct size range. Size heterogeneity can also result in different amounts of exosomal content. The microenvironment and the inherent biology of the cells may influence the content of the exosomes and their biological markers (Raghu Kalluri and Valerie S. LeBleu, Science Vol. 367, Issue 6478, 07 Feb 2020*).*

Disclosed herein are exosomes comprising a population of enriched exosomes in a concentration greater than about 1 × 10⁹ exosomes/mL. Also contemplated herein are methods for producing the disclosed exosomes.

The exosome composition can be derived from any number of tissue sources, such as muscle, fat, organ or bone or bone marrow. Various cells can be used to prepare the exosome composition of the present disclosure. Particularly, exosome composition can be derived from cells. In some embodiments of the disclosure, the cells are derived from a eukaryotic organism or a cell line. In some embodiments of the disclosure, the cells are derived from a plant or an animal. The cells may be genetically modified. In some embodiments of the disclosure, the cells are stem cells. In some embodiments, the stem cells are embryonic stem cells (ESCs), mesenchymal stem cells (MSCs), hematopoietic stem-cell transplantation (HSCT) or induced pluripotent stem cells (iPS cells or iPSCs).

A specific process is used to produce the exosome composition of the present disclosure. A desired amount of cells are provided firstly in a method for producing the exosome composition of the present disclosure. In some embodiments of the disclosure, the cells are cultured in a medium for a period of time sufficient to obtain a desired amount of cells. In some embodiments of the disclosure, the cell count is about 1 × 10⁵ cells/mL to about 1 × 10⁸ cells/mL; about 2 × 10⁵ cells/mL to about 8 × 10⁷ cells/mL; about 4 × 10⁵ cells/mL to about 6 × 10⁷ cells/mL; about 6 × 10⁵ cells/mL to about 4 × 10⁷ cells/mL; about 8 × 10⁵ cells/mL to about 2 × 10⁷ cells/mL; about 1 × 10⁶ cells/mL to about 1 × 10⁷ cells/mL; about 2 × 10⁶ cells/mL to about 8 × 10⁶ cells/mL; or about 4 × 10⁶ cells/mL to about 6 × 10⁶ cells/mL. The manners for culture depends on the cells. In some embodiments of the disclosure, the medium is a conditioned medium for obtaining the desired amount of cells with specific properties. For example, a conditioned medium is provided for maintaining cells at an undifferentiated stage or a differentiated stage.

The medium containing the desired amount of cells is subjected to a pre-clearance procedure for removing dead cells and/or cell debris. In one embodiment of the disclosure, the medium is centrifuged to remove dead cells. In some embodiments of the disclosure, dead cells can be removed at about 300 g, about 350 g, or about 400 g for about 5 minutes, about 6 minutes, about 7 minutes, about 8 minutes, about 9 minutes, about 10 minutes, about 11 minutes, about 12 minutes, about 13 minutes, about 14 minutes, or about 15 minutes. In one embodiment of the disclosure, the medium is centrifuged to remove cell debris. In some embodiments of the disclosure, cell debris can be removed at about 1500 g, about 1600 g, about 1700 g, about 1700 g, about 1800 g, about 1900 g, about 2000 g, about 2100 g, about 2200 g, about 2300 g, about 2400 g, or about 2500 g, for about 10 minutes, about 11 minutes, about 12 minutes, about 13 minutes, about 14 minutes, about 15 minutes, about 16 minutes, about 17 minutes, about 18 minutes, about 19 minutes, or about 20 minutes.

The resulting supernatant after removing the dead cells and/or cell debris is then filtrated to remove cell apoptotic bodies and microvesicles. In one embodiment of the disclosure, the resulting supernatant is filtrated by passing through about 0.15 µm, about 0.16 µm, about 0.18 µm, about 0.20 µm, about 0.22 µm, about 0.24 µm, about 0.26 µm, or about 0.28 µm filter.

In one embodiment of the disclosure, the resulting supernatant after pre-clearance is then subjected to ultrafiltration with a membrane having a molecular weight cutoff of about 3 kDa to about 100 kDa. In some embodiments of the disclosure, the ultrafiltration is performed via concentrator spin columns. In some embodiments of the disclosure, the membrane having a molecular weight cutoff of about 3 kDa, and the ultrafiltration is conducted for 480 minutes. In some embodiments of the disclosure, the membrane having a molecular weight cutoff of about 10 kDa, and the ultrafiltration is conducted for 60 minutes.

A type of membrane filtration in which forces (such as pressure or concentration gradients) lead to a separation through a semipermeable membrane. Ultrafiltration membranes are typically characterized by the molecular weight cut off of the membrane. Suspended solids and solutes of higher molecular weight are retained in the retentate, while water and lower molecular weight solutes pass through the membrane in the permeate. Different types of modules can be used for ultrafiltration processes. Examples of such modules are tubular elements that use polymeric membranes cast on the inside of plastic or paper tubes; hollow fiber designs that contain multiple hollow fibers; spiral wound modules in which flat membrane sheets are separated by a thin meshed spacer material that is rolled around a central perforated tube and fitted into a tubular steel pressure vessel casing; and plate and frame assemblies that use a membrane placed on a flat plate separated by a mesh like material through which the filtrate passes.

The resulting supernatant after ultrafiltration is then subjected to exosome precipitation for enrichment. In some embodiments of the disclosure, the resulting supernatant after ultrafiltration is precipitation with polymer-based precipitation. The resulting supernatant comprises a population of enriched exosomes in a concentration greater than about 1 × 10⁹ exosomes/mL.

Cell conditioned medium is a critical step for exosome extraction. Large amount of cell conditioned medium cause increase of cost for exosome extraction and enrichment. By ultrafiltration, cell conditioned medium is concentrated for enrichment of exosome extraction. The present disclosure provides an efficiency manufacture process of exosome enrichment for extraction.

In one embodiment of the disclosure, the resulting supernatant after pre-clearance is then subjected to ultracentrifugation at about 80,000 g to about 12,000 g for about 50 minutes to about 90 minutes.

The exosome composition can be identified by determination of its molecular weight, mean particle size, concentration of exosomes and range of particle size. The results are described herein. In some embodiments of the disclosure, in nanoparticle tracing analysis, exosome extraction after 3 kDa ultrafiltration shows to increase particle number compared to traditional ultracentrifugation. Exosome marker expression also increases in 3 kDa ultrafiltration. In addition, efficient of exosome extraction after 10 kDa ultrafiltration is similar to ultracentrifugation. Mean size is about 150 nm to about 160 nm, and size distribution is about 100 nm to about 200 nm.

The present exosome compositions may be used to treat any of the following diseases including, but not limited to: cancer and oncological disorders; infectious diseases; cardiovascular disease; diabetes mellitus including type-1 diabetes mellitus and type-2 diabetes mellitus; liver disease; obesity; rare diseases; gastro-intestinal diseases; skeletal diseases; and sickle cell disease. The present exosome compositions may also be used to cell therapeutics; vector and cell engineering, and pharmacology and toxicology assay development.

The present disclosure has been described herein using specific embodiments for the purposes of illustration only. It will be readily apparent to one of ordinary skill in the art, however, that the principles of the invention can be embodied in other ways. Therefore, the invention should not be regarded as limited in scope to the specific embodiments and claims.

### EXAMPLE

### Example 1 Production of An Exosome Composition

Umbilical cord mesenchymal stem cells (MSCs) were cultured in a cell conditioned medium for reaching the cell amout of 1.5 × 10⁶ cells/mL. The cultured medium was centrifuged at 350 g for 10 minutes at 4°C to remove dead cells. The resulting supernatant was further centrifuged at 2,000 g for 15 minutes at 4°C to remove the cell debris. The resulting supernatant was filtrated using a 0.22 µm filter to remove cell apoptotic bodies and microvesicles.

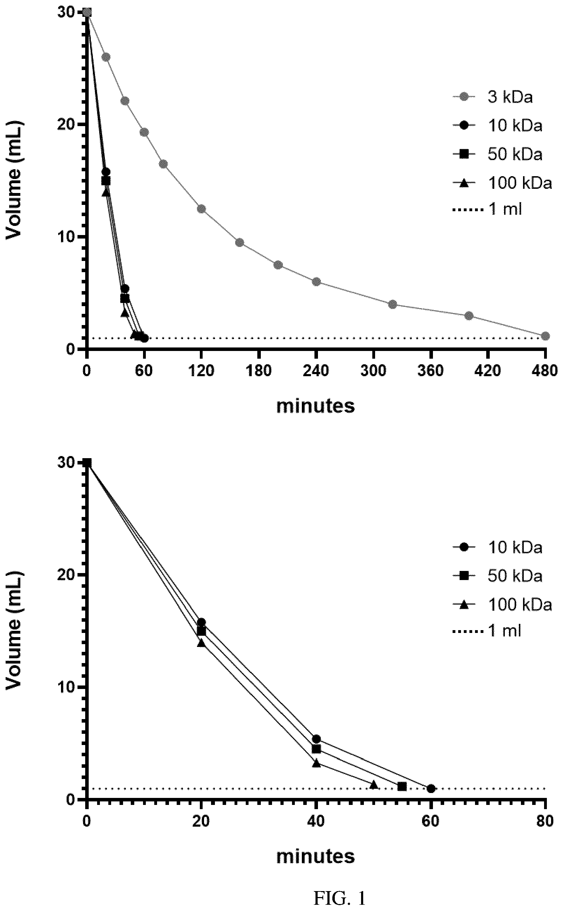

30 ml pre-cleared cell conditioned medium was ultrafiltrated via 3 kDa, 10 kDa, 50 kDa, and 100 kDa concentrator spin columns, VIVASPIN^{®} 6 (loading sample volume: 2 mL to 6 mL) and 20 (loading sample volume: 5 mL to 20 mL). Pore size of the membranes of the concentrator spin columns is proportional to kilo Dalton. Time consuming of 30 mL pre-cleared cell conditioned medium concentrating to 1 mL was estimated and shown in Table 1 and FIG. 1. As a result, 3 kDa ultrafiltration (480 minutes) took much time than 10 kDa (60 minutes), 50 kDa (55 minutes), 100 kDa (50 minutes).

**Table 1**

| Ultrafiltration (30 mL to 1 mL) | 3 kDa | 10 kDa | 50 kDa | 100 kDa |
|---|---|---|---|---|
| Pore size | 1.2 nm | 2.5 nm | 7 nm | 10 nm |
| Time (minutes) | 480 min | 60 min | 55 min | 50 min |

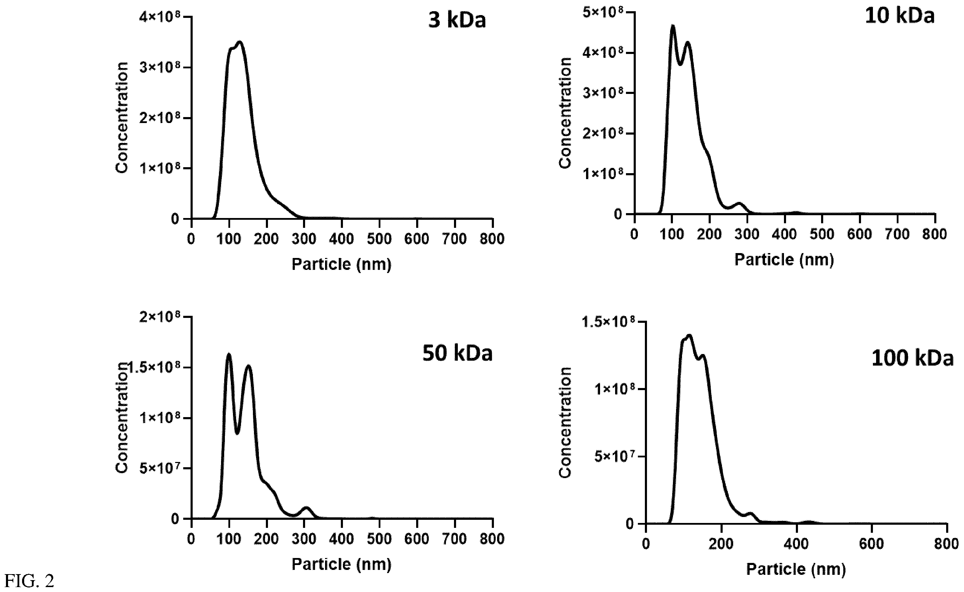

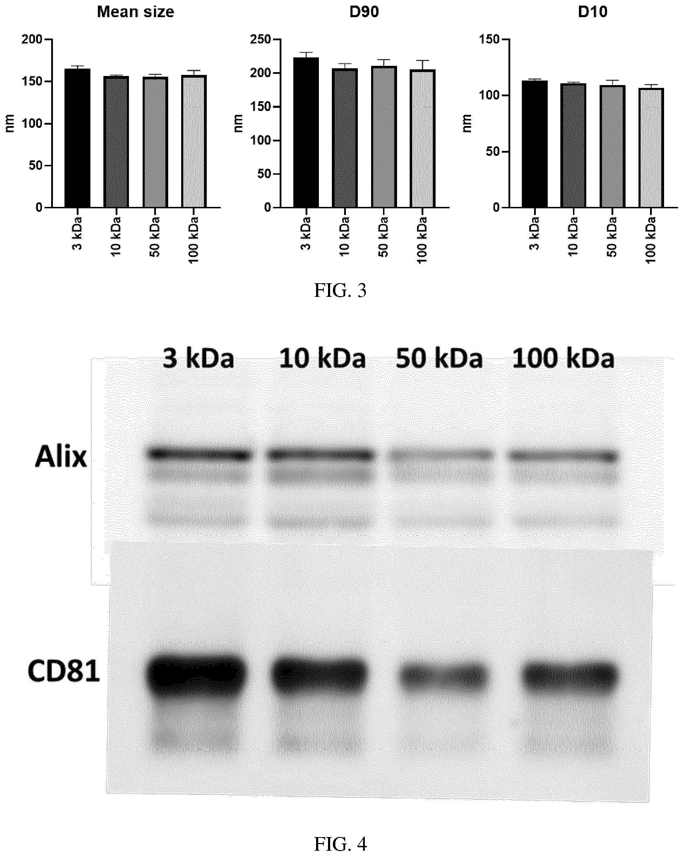

The exosomes were precipitated by polymer-based precipitation with TOOLSharp^{®} Cell Culture Media Exosome Extraction Kit. An exosome composition comprising a population of enriched exosomes was then obtained. The exosome composition was determined to have the characteristics in Table 2 and FIGs. 2 and 3. Size distribution of exosomes was analyzed by nanoparticle tracking analysis. As shown in FIG. 2, size distribution in 3 kDa, 10 kDa, 50 kDa, and 100 kDa was similar and in between 100 nm to 200 nm. As shown in FIG. 3, mean size is around 150 nm to 160 nm. D90 and D10 shown size distribution in between 100 nm to 200 nm.

**Table 2**

| | **3 kDa** | **10 kDa** | **50 kDa** | **100 kDa** |
|---|---|---|---|---|
| **CCM volume** | 30 mL | 30 mL | 30 mL | 30 mL |
| **Mean (Size)** | 139.7 nm | 143.7 nm | 145.0 nm | 142.2 nm |
| **D10** | 91.9 nm | 94.9 nm | 92.6 nm | 91.3 nm |
| **D90** | 191.8 nm | 197.8 nm | 205.9 nm | 197.5 nm |
| **Exosome Concentration** | 3.20 × 10¹⁰ | 4.26 × 10¹⁰ | 1.44 × 10¹⁰ | 1.48 × 10¹⁰ |
| **<200 nm** | 91.90 % | 90.71 % | 88.86 % | 90.93 % |
| **<100 nm** | 18.66 % | 16.27 % | 19.21 % | 18.70 % |

### Example 2 Exosome Composition Comprising a Population of Exosomes

The amounts of Alix and CD81 on the UCMSCs-derived exosomes obtained in Example 1 were measured by electrophoresis and shown in FIG. 4. Alix and CD81 decrease in 50 kDa and 100 kDa compared to 3 kDa.

Particle concentration decreased in 50 kDa and 100 kDa compared to 3 kDa via nanoparticle tracing analysis.

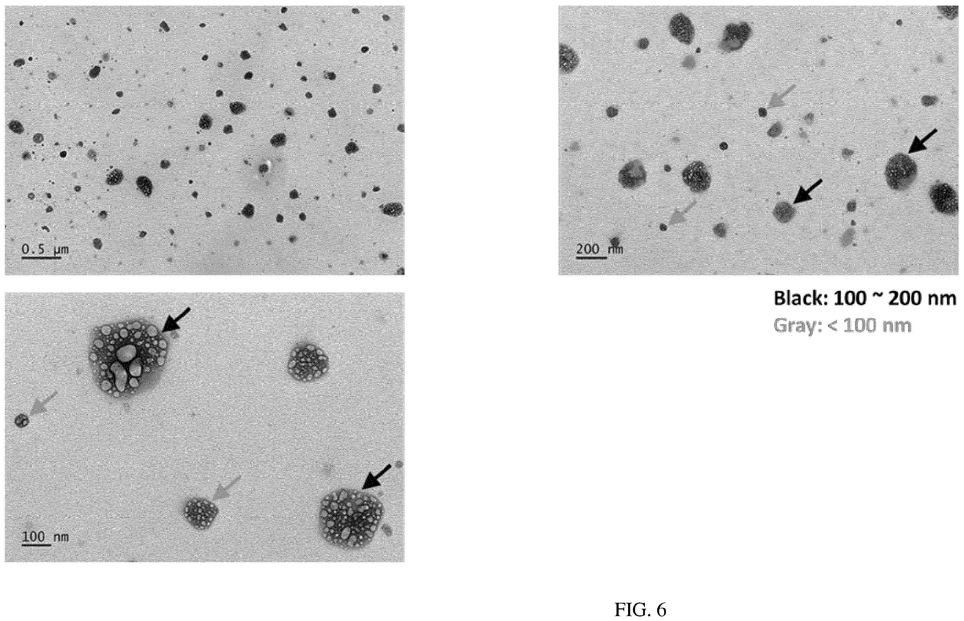

The transmission electron microscopy (TEM) analysis in UCMSCs-derived exosomes is shown in FIG. 6.

### Example 3 Production of An Exosome Composition-Ultracentrifugation

Umbilical cord mesenchymal stem cells (MSCs) were cultured in a cell conditioned medium for reaching the cell amout of 1.5 × 10⁶ cells/mL. The cultured medium was centrifuged at 350 g for 10 minutes at 4°C to remove dead cells. The resulting supernatant was further centrifuged at 2,000 g for 15 minutes at 4°C to remove the cell debris. The resulting supernatant was filtrated using a 0.22 µm filter to remove cell apoptotic bodies and microvesicles.

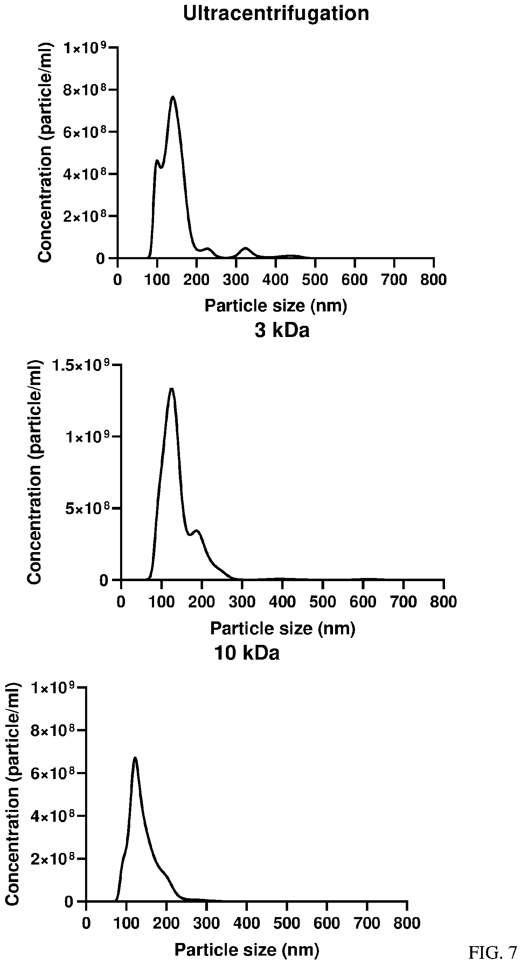

12 mL of the pre-cleared cell conditioned medium was ultracentrifugated at 100,000 g for 70 minutes. Size distributions of exosomes in this Example (ultracentrifugation) and Example 1 (3 kDa and 10 kDa) were analyzed by nanoparticle tracking analysis and shown in FIG. 7. The results show similar size distribution in between 100 nm to 200 nm.

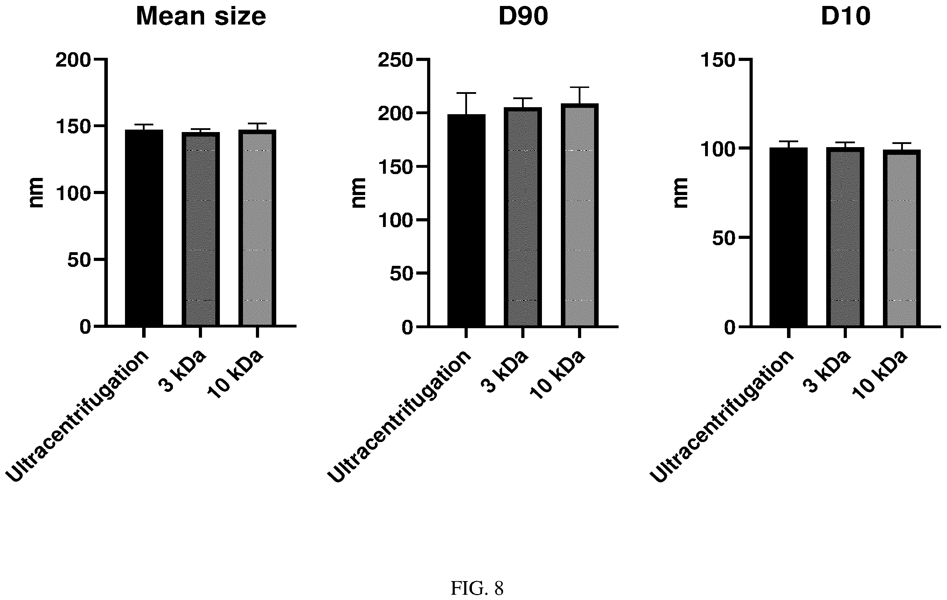

Parameter of nanoparticle tracking analysis in this Example (ultracentrifugation) and Example 1 (3 kDa and 10 kDa) were analyzed by nanoparticle tracking analysis and shown in FIG. 8. Mean sizes of all particles are around 150 nm to 160 nm. D90 and D10 shown size distribution in between 100 nm to 200 nm.

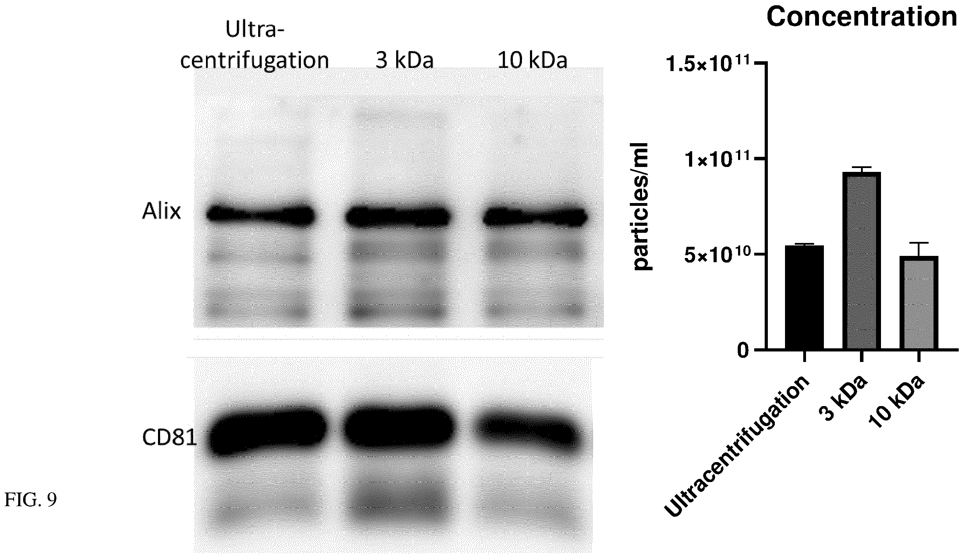

Particle concentration and the amounts of Alix and CD81 on the UCMSCs-derived exosomes obtained in in this Example (ultracentrifugation) and Example 1 (3 kDa and 10 kDa) were measured and shown in FIG. 9. Particle concentration increased in 3 kDa ultrafiltration compared to 10 kDa and ultracentrifugation. Alix and CD81 expression also increased in 3 kDa.

## Claims

1. An exosome composition comprising a population of enriched exosomes in a concentration greater than about 1 × 10⁹ exosomes/mL, wherein the exosome composition is directly obtained by isolation and ultrafiltration or ultracentrifugation rather than by formulation.

2. The exosome composition of claim 1, wherein the concentration of the exosomes in the composition is greater than about 1 × 10¹⁰ exosomes/mL.

3. The exosome composition of claim 1, wherein the exosome composition comprises a population of enriched exosomes having a mean particle size from about 135 nm to about 150 nm.

4. The exosome composition of claim 1, wherein the population of enriched exosomes comprises exosomes having molecular weight greater than about 3 kDa.

5. The exosome composition of claim 1, wherein the population of enriched exosomes comprises exosomes having molecular weight greater than about 10 kDa.

6. The exosome composition of claim 1, wherein the population of enriched exosomes comprises exosomes having molecular weight greater than about 50 kDa.

7. The exosome composition of claim 1, wherein the population of enriched exosomes comprises exosomes having molecular weight greater than about 100 kDa.

8. The exosome composition of claim 1, wherein the population of enriched exosomes comprises greater than about 80% exosomes having a particle size less than about 200 nm.

9. The exosome composition of claim 1, wherein the population of enriched exosomes comprises greater than about 10% exosomes having a particle size less than about 100 nm.

10. The exosome composition of claim 1, wherein the population of enriched exosomes comprises exosomes with a molecular weight greater than about 3 kDa, which have one or more of the following characteristics:
a concentration greater than about 1 × 10¹⁰ exosomes/mL;
about 90% to about 93% of exosomes having a particle size less than about 200 nm;
about 16% to about 20% of exosomes having a particle size less than about 100 nm; and
a mean particle size from about 135 nm to about 145 nm.

11. The exosome composition of claim 10, wherein the concentration is greater than about 2 × 10¹⁰ exosomes/mL; about 92% of exosomes have a particle size less than about 200 nm; about 17% to about 19% of exosomes have a particle size less than about 100 nm; and/or a mean particle size ranges from about 137 nm to about 142 nm.

12. The exosome composition of claim 1, wherein the population of enriched exosomes comprises exosomes with a molecular weight greater than about 10 kDa, which have one or more of the following characteristics:
a concentration greater than about 2 × 10¹⁰ exosomes/mL;
about 90% to about 93% of exosomes having a particle size less than about 200 nm;
about 14% to about 18% of exosomes having a particle size less than about 100 nm; and
a mean particle size from about 138 nm to about 148 nm.

13. The exosome composition of claim 12, wherein the concentration is greater than about 2.5 × 10¹⁰ exosomes/mL; about 91% of exosomes have a particle size less than about 200 nm; about 15% to about 17% of exosomes have a particle size less than about 100 nm; and/or a mean particle size ranges from about 140 nm to about 145 nm.

14. The exosome composition of claim 1, wherein the population of enriched exosomes comprises exosomes with a molecular weight greater than about 50 kDa, which have one or more of the following characteristics:
a concentration greater than 1.0 × 10¹⁰ exosomes/mL;
about 85% to about 90% of exosomes having a particle size less than about 200 nm;
about 15% to about 25% of exosomes having a particle size less than about 100 nm; and
a mean particle size from about 140 nm to about 150 nm.

15. The exosome composition of claim 14, wherein the concentration is greater than about 1.0 × 10¹⁰ exosomes/mL; about 88.86% of exosomes have a particle size less than about 200 nm; about 18% to about 20% of exosomes have a particle size less than about 100 nm; and/or a mean particle size ranges from about 142 nm to about 148 nm.

16. The exosome composition of claim 1, wherein the population of enriched exosomes comprises exosomes with a molecular weight greater than about 100 kDa, which have one or more of the following characteristics:
a concentration greater than 1.0 × 10¹⁰ exosomes/mL;
about 88% to about 92% of exosomes having a particle size less than about 200 nm;
about 15% to about 20% of exosomes having a particle size less than about 100 nm; and
a mean particle size from about 138 nm to about 145 nm.

17. The exosome composition of claim 16, wherein the concentration is greater than about 1.2 × 10¹⁰ exosomes/mL; about 90.93% of exosomes have a particle size less than about 200 nm; about 16% to about 19% of exosomes have a particle size less than about 100 nm; and/or a mean particle size ranges from about 140 nm to about 144 nm.

18. The exosome composition of claim 1, wherein the exosomes are derived from stem cells.

19. A method for producing the exosome composition of claim 1, comprising the steps of:
providing a desired amount of cells in a medium;
centrifuging the medium to remove cell debris and then filtrating the resulting supernatant to remove cell apoptotic bodies and microvesicles;
ultrafiltrating the resulting supernatant with a membrane having a molecular weight cutoff of about 3 kDa to about 100 kDa; and
precipitating exosomes with polymer-based precipitation through centrifuging the resulting supernatant at about 6,000 g to about 15,000 g for at least about 10 minutes to obtain the exosome composition comprising enriched exosomes.

20. A method for producing the exosome composition of claim 1, comprising the steps of:
providing a desired amount of cells in a medium;
centrifuging the medium to remove cell debris and then filtrating the resulting supernatant to remove cell apoptotic bodies and microvesicles;
ultracentrifugating the resulting supernatant at about 80,000 g to about 12,000 g for about 50 minutes to about 90 minutes to obtain the exosome composition comprising enriched exosomes.
